# EUROPÄISCHE PATENTANMELDUNG

(11) **EP 3 940 713 A1**
(43) Veröffentlichungstag der Anmeldung: **19.01.2022**
(21) Anmeldenummer: 21185129.0
(22) Anmeldetag: 12.07.2021
(51) Int. Cl.: G16H 40/63, G16H 40/67

(54) **FERNSTEUERUNG VON RADIOLOGISCHEN UNTERSUCHUNGEN**

(30) Priorität: 13.07.2020 DE 102020208715
(71) Anmelder: MEDiCI GmbH, 81245 München (DE)
(72) Erfinder: ENGLMAIER, Malik, 84559 Kraiburg (DE); REIHER, Michael, 72762 Reutlingen (DE)
(74) Vertreter: Bardehle Pagenberg Partnerschaft mbB Patentanwälte Rechtsanwälte

(57) **Zusammenfassung**

Die vorliegende Erfindung betrifft ein System zur Durchführung einer bildgebenden diagnostischen Untersuchung, mit einer Untersuchungsvorrichtung, insbesondere einer MRT- oder CT-Untersuchungsvorrichtung, eingerichtet zur Erzeugung von diagnostischen Bilddaten, einer lokalen Bedienvorrichtung, eingerichtet zur Steuerung der Untersuchungsvorrichtung, und einer externen Bedienvorrichtung, die separat zu der lokalen Bedienvorrichtung bereitgestellt ist.

## Beschreibung

### 1. Technisches Gebiet

Die vorliegende Erfindung betrifft ein System zur Durchführung einer bildgebenden diagnostischen Untersuchung, als auch ein entsprechendes Verfahren. Die Erfindung betrifft insbesondere die Durchführung einer bildgebenden diagnostischen Untersuchung mittels einer lokalen Bedienvorrichtung und einer externen Bedienvorrichtung, die separat zu der lokalen Bedienvorrichtung bereitgestellt ist.

### 2. Technischer Hintergrund

In der medizinischen Diagnostik werden bildgebende radiologische Verfahren eingesetzt, wie etwa Magnetresonanztomographie (MRT), Computertomographie (CT) oder Radiographie, um beispielsweise die Struktur und Funktion von Gewebe und Organen eines Patienten visuell darzustellen. Hierzu werden bildgebende Untersuchungsvorrichtungen verwendet, die eine entsprechende bilderzeugende Modalität umfassen können.

Zur Steuerung der Untersuchungsvorrichtungen ist üblicherweise eine Bedienkonsole vorgesehen, welche entweder integral oder im näheren Umfeld mit der jeweiligen bilderzeugenden Modalität bereitgestellt ist. Beispielsweise kann sich die Untersuchungsvorrichtung in einem Untersuchungsraum befinden, und die Bedienkonsole in einem Vorraum des Untersuchungsraums. Im Rahmen einer Untersuchung wird der Patient üblicherweise von einer spezialisieren Fachkraft für die Untersuchung vorbereitet, und in dem Untersuchungsraum an der Untersuchungsvorrichtung positioniert. Ferner kann die spezialisiere Fachkraft an der Bedienkonsole die Patientendaten laden und ein Untersuchungsprogramm auswählen, abhängig von der jeweiligen diagnostischen Fragestellung. Im Anschluss startet die spezialisiere Fachkraft das Untersuchungsprogramm von der Bedienkonsole aus, und bewertet sodann die von der Untersuchungsvorrichtung an die Bedienkonsole ausgegebenen Bilddaten. Nach Abschluss der Untersuchung wird der Patient üblicherweise von der spezialisierten Fachkraft nachversorgt.

Die spezialisierte Fachkraft muss somit neben der eigentlichen Durchführung des Untersuchungsprogramms eine Vielzahl von weiteren Tätigkeiten durchführen, die auch eine allgemeine Hilfskraft durchführen kann, wodurch letztlich auch der Einsatz der spezialisierten Fachkraft selbst ineffizient wird.

Die Druckschrift DE 10 2012 200 593 A1 betrifft ein Befundungssystem, ein Steuerungssystem und ein computer-implementiertes Verfahren zur Befundung von medizinischen Bildern. Dabei wird ein Akquisitionssystem offenbart, umfassend eine Bildakquisitionseinrichtung und eine Konsole, über welche die Bildakquisitionseinrichtung gesteuert wird. Ferner wird ein von dem Akquisitionssystem entkoppeltes Befundungssystem zur Befundung von medizinischen Bilddaten offenbart, umfassend einen Befundungscomputer mit einer Monitoreinheit zur Darstellung der Bilddaten. Das Befundungssystem umfasst dabei ferner ein Steuermodul, welches im Rahmen eines Befundungsworkflows aktivierbar ist und ferner dazu bestimmt ist, lokal auf dem Befundungssystem eingegebene Steuerdaten zu erfassen und diese automatisch in Steuerbefehle zu konvertieren, die dazu ausgebildet sind, direkt und automatisch über eine Schnittstelle an das Akquisitionssystem übertragen zu werden. Von dem Befundungssystem können spezifische Steuerbefehle wie etwa eine Repeat-Funktion und/oder eine Append-Funktion aus aufgerufen werden. Es kann somit lediglich nach der Bildakquisition spezielle Funktionen durchgeführt werden. Dieses System erweist sich als unflexibel.

Aufgabe der vorliegenden Erfindung ist es, ein effizienteres Durchführen einer bildgebenden diagnostischen Untersuchung zu ermöglichen.

Diese Aufgabe wird vorliegend durch ein System gemäß Anspruch 1 und ein Verfahren gemäß Anspruch 12 gelöst. Weitere Ausgestaltungen sind durch die Unteransprüche beschrieben.

### 3. Inhalt der Erfindung

Ein Aspekt der Erfindung betrifft ein System zur Durchführung einer bildgebenden diagnostischen Untersuchung. Beispielsweise kann mit diesem System ein radiologisches Verfahren durchgeführt werden, wie etwa MRT oder CT. Das System umfasst hierzu eine Untersuchungsvorrichtung, wie beispielsweise eine MRT- oder CT-Untersuchungsvorrichtung, die zur Erzeugung diagnostischer Bilddaten eingerichtet ist. Hierzu kann die Untersuchungsvorrichtung eine entsprechende bildgebende Modalität umfassen.

Das System umfasst ferner eine lokale Bedienvorrichtung. Diese lokale Bedienvorrichtung kann dabei zumindest teilweise integral mit der Untersuchungsvorrichtung ausgebildet sein, und/oder sich zumindest teilweise in näherer Umgebung wie etwa in einem benachbarten Raum oder im gleichen Gebäude zu der Untersuchungsvorrichtung befinden. Die lokale Bedienvorrichtung ist dabei eingerichtet, um die Untersuchungsvorrichtung zu steuern.

Hierzu umfasst die lokale Bedienvorrichtung eine lokale Eingabeeinheit, die eingerichtet ist, um Nutzereingaben zu registrieren. Beispielsweise kann die lokale Eingabeeinheit eine Tastatur, eine Maus, und/oder einen Joystick umfassen. Über diese lokale Eingabeeinheit hat ein Nutzer die Möglichkeit, Eingaben zu tätigen, um letztlich die Untersuchungsvorrichtung zu steuern. Die lokale Bedienvorrichtung umfasst ferner eine lokale grafische Ausgabeeinheit, die eingerichtet ist, um die mit der Untersuchungsvorrichtung erzeugten Bilddaten darzustellen. Beispielsweise kann die lokale grafische Ausgabeeinheit einen Monitor umfassen, über welchen der Nutzer die erzeugten Bilddaten einsehen kann. Ferner umfasst die lokale Bedienvorrichtung Steuermittel zur Steuerung der Untersuchungsvorrichtung. Beispielsweise können die Steuermittel eine Recheneinheit umfassen, welche direkt oder indirekt, beispielsweise netzwerkbasiert, mit der Untersuchungsvorrichtung verbunden ist und mit dieser kommuniziert. Die lokale Eingabeeinheit und die lokale grafische Ausgabeeinheit sind dabei vorzugsweise mit den Steuermittels verbunden, können aber auch zumindest teilweise integral mit diesen ausgestaltet sein.

Das System umfasst ferner eine externe Bedienvorrichtung. Diese externe Bedienvorrichtung ist dabei separat zu der lokalen Bedienvorrichtung bereitgestellt. Die externe Bedienvorrichtung kann dabei ebenfalls in der näheren Umgebung zu der Untersuchungsvorrichtung angeordnet sein, ist bevorzugt aber weiter entfernt als die lokale Bedienvorrichtung von der Untersuchungsvorrichtung vorgesehen. Beispielsweise kann sich die externe Bedienvorrichtung in einem anderen Gebäude befinden. Die externe Bedienvorrichtung kann dabei über eine echtzeitfähige Datenverbindung mit der lokalen Bedienvorrichtung verbunden sein.

Die externe Bedienvorrichtung umfasst eine externe Eingabeeinheit, die eingerichtet ist, um Nutzereingaben zu registrieren. Beispielsweise kann die lokale Eingabeeinheit eine Tastatur, eine Maus, und/oder einen Joystick umfassen. Über diese lokale Eingabeeinheit hat ein Nutzer die Möglichkeit, Eingaben zu tätigen, um letztlich die Untersuchungsvorrichtung zu steuern. Die lokale Bedienvorrichtung umfasst ferner eine lokale grafische Ausgabeeinheit, die eingerichtet ist, um die mit der Untersuchungsvorrichtung erzeugten Bilddaten darzustellen. Beispielsweise kann die lokale grafische Ausgabeeinheit einen Monitor umfassen, über welchen der Nutzer die erzeugten Bilddaten einsehen kann.

Das System und insbesondere die Steuermittel zur Steuerung der Untersuchungsvorrichtung sind dabei eingerichtet, um die Untersuchungsvorrichtung gemäß den durch die lokale Eingabeeinheit registrierten Nutzereingaben zu steuern. Das System und insbesondere die Steuermittel sind ferner eingerichtet, um die Untersuchungsvorrichtung gemäß den durch die externe Eingabeeinheit registrierten Nutzereingaben zu steuern. Somit kann die Untersuchungsvorrichtung sowohl über die lokale Eingabeeinheit gesteuert werden als auch über die externe Eingabeeinheit. Ferner ist das System und insbesondere die Steuermittel eingerichtet, um die Untersuchungsvorrichtung gemäß den durch die lokale und externe Eingabeeinheit registrierten Nutzereingaben in Echtzeit zu steuern. Somit kann ohne Verzögerung, abgesehen von durch die Kommunikationswege auftretende Latenz, die Untersuchungsvorrichtung von der externen Stelle aus gesteuert werden, ohne dass eine spezialisierte Fachkraft zwingend an der lokalen Bedienvorrichtung Eingaben tätigen muss.

Das System ermöglicht es somit einer spezialisierten Fachkraft, das Untersuchungsprogramm vollständig von der externen Bedienvorrichtung aus zu steuern. Es ist nicht notwendig, dass sich die spezialisierte Fachkraft in näherer Umgebung zu dem Patienten und der Untersuchungsvorrichtung befindet. Die Betreuung des Patienten vor Ort kann von einer allgemeinen Hilfskraft durchgeführt werden, die nicht über spezielle Fachkenntnisse hinsichtlich der jeweiligen diagnostischen Fragestellung verfügen muss, und das Untersuchungsprogramm nicht steuern oder anpassen muss. Der Einsatz der spezialisierten Fachkraft wird somit effizienter gestaltet.

Hierzu umfassen die lokale Bedienvorrichtung eine lokale Verbindungseinheit, und die externe Bedienvorrichtung eine entsprechende externe Verbindungseinheit. Diese Verbindungseinheiten können Hardwaremodule umfassen, und/oder zumindest teilweise durch Softwaremodule (z.B. einer App) ausgebildet sein. Die lokale Verbindungseinheit und die externe Verbindungseinheit sind dabei zur Kommunikation miteinander über ein Netzwerk eingerichtet. Die Kommunikation kann dabei über ein Local Area Network (LAN) und/oder ein Wide Area Network (WAN) erfolgen, vorzugsweise gesichert. Vorzugsweise kann die Kommunikation über das Internet erfolgen. Vorzugsweise erfolgt die Kommunikation verschlüsselt. Insbesondere, wenn die Kommunikation über das Interner erfolgt, erfolgt diese vorzugsweise über eine verschlüsselte Datenverbindung. Die externe Bedienvorrichtung kann somit mittels der Verbindungseinheiten mit der lokalen Bedienvorrichtung, und insbesondere mit den Steuermitteln der lokalen Bedienvorrichtung kommunizieren. Hierdurch wird eine effiziente Fernsteuerung der Untersuchungsvorrichtung von der externen Bedienvorrichtung aus möglich. Eine externe Fachkraft kann sich somit an einem externen Arbeitsplatz befinden, und direkt bei Bedarf die Steuerung über die Untersuchungsvorrichtung übernehmen. Zusätzlich oder alternativ wird die vollständige Steuerung auch von der lokalen Bedienvorrichtung aus ermöglicht. Durch Installation der Verbindungseinheiten kann ein bestehendes System mit geringem Aufwand für die Steuerung durch die externe Bedienvorrichtung nachgerüstet werden. Die Konfiguration der Steuermittel muss dabei nicht geändert werden.

Vorzugsweise umfasst die lokale Verbindungseinheit und die externe Verbindungseinheit jeweils ein KVM-Verlängerungsmodul. Ein KVM-Verlängerungsmodul (KVM: "Keyboard Video Mouse") bietet die Möglichkeit, mittels einer separaten oder externen Konsole umfassend Tastatur, Maus und Monitor einen lokalen Rechner zu steuern. Somit kann zusätzlich auch von der separaten oder externen Konsole aus der lokale Rechner bedient werden. Im vorliegenden Fall ist es somit möglich, die Steuermittel und entsprechend die Untersuchungsvorrichtung mittels der externen Eingabeeinheit zu bedienen, wobei die entsprechenden Steuersignale mittels der KVM-Verlängerungsmodule übertragen werden. Vorzugsweise sind die KVM-Verlängerungsmodule zur IP-fähigen Kommunikation über das Internet eingerichtet. Die lokale Verbindungseinheit kann dabei ein IP-basierter KVM-Verlängerungssender ("*KVM Extender Transmitter")* sein, und die externe Verbindungseinheit ein IP-basierter KVM-Verlängerungsempfänger ("*KVM Extender Receiver"*). Die Einrichtung des Systems kann somit mit minimalem Aufwand erfolgen. An der lokalen Bedienvorrichtung muss lediglich die entsprechende lokale Verbindungseinheit mit den Steuermitteln verbunden werden, und die hinzuzufügende externe Bedienvorrichtung muss lediglich mit der entsprechenden externen Verbindungseinheit und den gewünschten externen Eingabeeinheiten ausgestattet werden. Somit können bestehende Systeme ohne aufwendige Konfigurationen mit geringem Aufwand nachgerüstet werden. Insbesondere sind keine weiteren Konfigurationen an den Steuermitteln für die Nachrüstung notwendig.

In einer bevorzugten Ausführungsform kann die lokale Verbindungseinheit ein im Wesentlichen durch Hardware ausgebildeter Sender sein, wie etwa ein IP-basierter KVM-Verlängerungssender, und die externe Verbindungseinheit kann ein im Wesentlichen durch Software ausgebildeter Empfänger sein, wie etwa eine App, die über ein Netzwerk mit dem Sender kommunizieren kann um somit eine externe Steuerung zu ermöglichen. Hierbei kann die externe Bedienvorrichtung beispielsweise durch ein integrales Gerät ausgebildet sein, wie etwa ein Benutzerendgerät, beispielsweise ein Tabletcomputer. Die externe Bedienvorrichtung kann auch ein Desktop-Computer sein, in welchem die externe Verbindungseinheit durch ein Hardware und/oder Softwaremodul implementiert sein kann.

Die lokale Verbindungseinheit und die externe Verbindungseinheit sind vorzugsweise eingerichtet, um die durch die externe Eingabeeinheit registrierten Nutzereingaben an die Steuermittel zu übertragen. Ferner sind die lokale und externe Verbindungseinheit vorzugsweise eingerichtet, um die mit der Untersuchungsvorrichtung erzeugten Bilddaten an die externe grafische Ausgabeeinheit zu übertragen. Dadurch kann die externe Bedienvorrichtung an einem beliebigen Ort vorliegen und zur vollständigen Steuerung der Untersuchungsvorrichtung verwendet werden. Insbesondere kann die externe Eingabeeinheit und die externe grafische Ausgabeeinheit der externen Bedienvorrichtung direkt mit der externen Verbindungseinheit verbunden sein. Beispielsweise kann eine Tastatur und eine Maus direkt an die externe Verbindungseinheit angeschlossen werden, etwa über eine USB-Verbindung. Damit kann die externe Bedienvorrichtung mit geringem Aufwand eingerichtet werden.

Vorzugsweise umfasst die lokale Bedienvorrichtung einen Videosplitter. Der Videosplitter ist dabei eingerichtet, um eingehende Videosignale auf die lokale grafische Ausgabeeinheit und (über die Verbindungseinheiten) auf die externe grafische Ausgabeeinheit zu splitten. Der Videosplitter ist dabei eingerichtet um ein eingehendes Videosignal zu splitten, wobei das gesplittete Videosignal an die lokale grafische Ausgabeeinheit und über die lokale Verbindungseinheit und die externe Verbindungseinheit an die externe grafische Ausgabeeinheit ausgegeben wird. Somit werden die (von den Steuermitteln) eingehenden Videosignale, welche beispielsweise die von der Untersuchungsvorrichtung erzeugten Bilddaten umfassen, gleichzeitig an der lokalen Bedienvorrichtung und an der externen Bedienvorrichtung dargestellt. Der Videosplitter kann hierzu mit einem entsprechenden Videoausgang der Steuermittel, und mit entsprechenden Videoeingängen der grafischen Ausgabeeinheiten direkt oder indirekt verbunden sein. Beispielsweise kann der Videosplitter direkt über ein entsprechendes Kabel mit der lokalen grafischen Ausgabeeinheit verbunden sein, und indirekt über die Verbindungseinheiten und das Netzwerk mit der externen grafischen Ausgabeeinheit. In einem bevorzugten Ausführungsbeispiel ist der Eingang des Videosplitters mit dem Steuermittel (z.B. Steuerrechner) verbunden, ein erster Ausgang mit der lokalen Ausgabeeinheit, und ein zweiter Ausgang mit der lokalen Verbindungseinheit. Dies vereinfacht die Einrichtung der externen Bedienvorrichtung, da lediglich neben den Verbindungseinheiten der Videosplitter verbunden werden muss, um die Eingabe- und Anzeige- bzw. Darstellungsfunktionen der lokalen Bedienvorrichtung an die externe Bedienvorrichtung zu spiegeln.

Vorzugsweise sind die Steuermittel zumindest teilweise durch eine Recheneinheit ausgebildet. Diese ist dazu eingerichtet, Steuersignale gemäß den registrierten Nutzereingaben an die Untersuchungsvorrichtung auszugeben. In der Recheneinheit können Programmmodule gespeichert sein, welche durch die Nutzereingaben aktiviert werden können, um ein Untersuchungsprogramm mit der Untersuchungsvorrichtung zu starten. Die Recheneinheit ist ferner dazu eingerichtet, die von der Untersuchungsvorrichtung erzeugten diagnostischen Bilddaten zu empfangen, und diese auszugeben. Beispielsweise können von der Untersuchungsvorrichtung eingehende Bildsignale durch die Recheneinheit aufbereitet werden, und anschließend zur grafischen Darstellung an die Ausgabeeinheiten ausgegeben werden. In einer bevorzugten Ausführungsform kann die Ausgabe der Bilddaten über einen Videosplitter erfolgen, welcher integral mit der Recheneinheit ausgebildet sein kann.

Insbesondere vorzugsweise ist die Recheneinheit eingerichtet, um über die externe Eingabeeinheit unabhängig von der lokalen Eingabeeinheit bedient zu werden, und um über die lokale Eingabeeinheit unabhängig von der externen Eingabeeinheit bedient zu werden. Die vollständige Steuerung der Untersuchungsvorrichtung kann dabei über die lokale Eingabeeinheit erfolgen, und/oder über die externe Eingabeeinheit. Es ist somit nicht erforderlich, dass ein Nutzer an der lokalen Bedienvorrichtung Eingaben tätigt. Die spezialisierte Fachkraft kann somit effizient von einem externen Arbeitsplatz aus arbeiten. Die Versorgung des Patienten vor Ort kann durch eine allgemeine Hilfskraft bewerkstelligt werden.

Insbesondere vorzugsweise ist die Recheneinheit eingerichtet, um ab Bootvorgang über die externe Eingabeeinheit und über die lokale Eingabeeinheit bedient zu werden. Der Nutzer an der externen Bedienvorrichtung hat somit die vollständige Kontrolle über die Recheneinheit. Es ist somit nicht erforderlich, dass ein Nutzer an der lokalen Bedienvorrichtung Eingaben tätigt. Vorzugsweise kann die Recheneinheit durch die externe Bedienvorrichtung bzw. mittels der externen Eingabeeinheit eingeschaltet und hochgefahren werden. Somit kann die externe Fachkraft bei Bedarf von einem externen Arbeitsplatz aus entsprechende Untersuchungsprogramme autonom und plattformunabhängig aktivieren. Die allgemeine Hilfskraft vor Ort muss sich nur um den Patienten kümmern, und gegebenenfalls lediglich die Recheneinheit einschalten.

Vorzugsweise ist das System dazu eingerichtet, um eine Steuerung der Untersuchungsvorrichtung mittels der externen Bedienvorrichtung vor, während und nach der Erzeugung der diagnostischen Bilddaten zu ermöglichen. Somit kann der Nutzer über die externe Bedienvorrichtung jederzeit die Untersuchungsvorrichtung steuern. Beispielsweise kann der Nutzer über die externe Bedienvorrichtung während der Bilderzeugung erkennen, dass Änderungen des Untersuchungsprogramms vorzunehmen sind, und diese direkt durchführen. Die spezialisierte Fachkraft kann beispielsweise während des Ablaufs des Untersuchungsprogramms Änderungen an diesem vornehmen, um die Resultate zu optimieren. Beispielsweise kann die spezialisierte Fachkraft erkennen, dass die Untersuchung auf eine bestimmte Region des Körpers des Patienten fokussiert werden sollte, und das Untersuchungsprogramm entsprechend abändern. Das System erlaubt es der spezialisierten Fachkraft direkt in die ablaufende Untersuchung entsprechend einzugreifen.

In einer weiteren Ausführungsform umfasst das System eine zweite Untersuchungsvorrichtung, welche ebenfalls zur Erzeugung von diagnostischen Bilddaten eingerichtet ist. Die zweite Untersuchungsvorrichtung kann dabei der zuvor beschriebenen (ersten) Untersuchungsvorrichtung entsprechen, kann aber auch nach einem anderen Verfahren (z.B. MRT oder CT) arbeiten und auch von einem anderen Hersteller als die zuvor beschriebenen (ersten) Untersuchungsvorrichtung stammen. Die zweite Untersuchungsvorrichtung kann sich an einem separaten Ort befinden, beispielsweise in einem anderen Gebäude (möglicherweise einer anderen medizinischen Einrichtung) als die erste Untersuchungsvorrichtung. Ferner umfasst das System eine zweite lokale Bedienvorrichtung, welche zur Steuerung der zweiten Untersuchungsvorrichtung eingerichtet ist. Die zweite lokale Bedienvorrichtung kann integral oder in der Nähe der zweiten Untersuchungsvorrichtung bereitgestellt sein. Die zweite lokale Bedienvorrichtung kann dabei der zuvor beschriebenen (ersten) lokalen Bedienvorrichtung entsprechen, und kann Komponenten eines anderen Herstellers umfassen. Die zweite lokale Bedienvorrichtung umfasst somit entsprechend eine zweite lokale Eingabeeinheit, die zur Registrierung von Nutzereingaben eingerichtet ist, eine zweite lokale grafische Ausgabeeinheit, die zur Darstellung der mit der zweiten Untersuchungsvorrichtung erzeugten Bilddaten eingerichtet ist, und zweite Steuermittel zur Steuerung der zweiten Untersuchungsvorrichtung. Die zweite Bedienvorrichtung umfasst ferne eine entsprechende zweite lokale Verbindungseinheit, die zur Kommunikation mit der externen Verbindungseinheit der externen Bedienvorrichtung über das Netzwerk eingerichtet ist. Das System und insbesondere die zweiten Steuermittel sind dabei eingerichtet, um die zweite Untersuchungsvorrichtung gemäß den durch die zweite lokale Eingabeeinheit registrierten Nutzereingaben zu steuern, und um die zweite Untersuchungsvorrichtung gemäß den durch die externe Eingabeeinheit registrierten Nutzereingaben zu steuern, wobei die Steuerung mittels der Eingabeeinheiten in Echtzeit erfolgt. Es ist somit eine externe Bedienvorrichtung vorgesehen, mittels welcher eine spezialisierte Fachkraft mehrere (auch unterschiedliche) Untersuchungsvorrichtungen steuern kann. Hierdurch kann der Einsatz der spezialisierten Fachkraft optimiert werden. Dies erfolgt unabhängig vom Hersteller der Untersuchungsvorrichtungen. Der Fachmann versteht, dass hierbei ein KVM-Verlängerung-und-Switching-System eingesetzt werden kann, um die Steuerbefehle von der externen Steuereinheit an die jeweilige lokale Bedienvorrichtung bzw. die entsprechende Untersuchungsvorrichtung zu leiten.

Ein weiterer Aspekt der Erfindung betrifft ein Verfahren zur Durchführung einer bildgebenden diagnostischen Untersuchung. Das Verfahren umfasst dabei ein Bereitstellen eines Systems gemäß den vorstehenden Ausführungen. Es wird somit ein System zur Durchführung einer bildgebenden diagnostischen Untersuchung bereitgestellt, umfassend gemäß den vorstehenden Ausführungen zumindest eine Untersuchungsvorrichtung, eine lokale Bedienvorrichtung, und eine externe Bedienvorrichtung.

Das Verfahren umfasst ferner ein Herstellen einer Kommunikation zwischen der lokalen Bedienvorrichtung und der externen Bedienvorrichtung. Hierzu kann beispielsweise eine IP-fähige Kommunikation über das Internet mittels der Verbindungseinheiten hergestellt werden. Vorzugsweise kann die Kommunikation über eine verschlüsselte Datenverbindung erfolgen.

Das Verfahren umfasst ferner ein Darstellen von Patientendaten auf der lokalen grafischen Ausgabeeinheit und auf der externen grafischen Ausgabeeinheit. Die Patientendaten können dabei aus einer Datenbank geladen werden, auf welche die Steuermittel zugreifen kann. Die Steuermittel können die entsprechenden Grafiksignale (direkt oder indirekt) an die grafischen Ausgabeeinheiten ausgeben.

Das Verfahren umfasst ferner ein Ansteuern der Untersuchungsvorrichtung zum Durchführen eines Untersuchungsprogramms zur Erzeugung von diagnostischen Bilddaten. Dies erfolgt basierend auf durch die lokale Eingabeeinheit und/oder durch die externe Eingabeeinheit registrierten Nutzereingaben, die über die Verbindungseinheiten an die Steuermittel kommuniziert werden können. So kann eine spezialisierte Fachkraft über die externe Eingabeeinheit von der externen Bedienvorrichtung aus das Untersuchungsprogramm autonom starten.

Das Verfahren umfasst ferner, während des Durchführens des Untersuchungsprogramms, ein Darstellen von erzeugten diagnostischen Bilddaten auf der lokalen grafischen Ausgabeeinheit und auf der externen grafischen Ausgabeeinheit. Die spezialisierte Fachkraft kann somit über die externe grafische Ausgabeeinheit erste Ergebnisse der Untersuchung erkennen. Hierzu können die Steuermittel entsprechende Grafiksignale (direkt oder indirekt) an die grafischen Ausgabeeinheiten ausgeben.

Das Verfahren umfasst ferner im (unmittelbaren oder mittelbaren) Anschluss an den vorangegangenen Schritt des Anzeigens, während des Durchführens des Untersuchungsprogramms, ein Anpassen des Untersuchungsprogramms. Dies erfolgt basierend auf durch die lokale Eingabeeinheit und/oder durch die externe Eingabeeinheit registrierten Nutzereingaben, die über die Verbindungseinheiten an die Steuermittel kommuniziert werden können. Daraufhin wird die Untersuchung basierend auf dem angepassten Untersuchungsprogramm fortgesetzt. Die spezialisierte Fachkraft kann somit das Untersuchungsprogramm bedarfsgerecht anpassen, noch während das Untersuchungsprogramm läuft.

Das Verfahren umfasst ferner, während des Durchführens des angepassten Untersuchungsprogramms, ein Darstellen von erzeugten diagnostischen Bilddaten auf der lokalen grafischen Ausgabeeinheit und auf der externen grafischen Ausgabeeinheit. Hierzu können die Steuermittel entsprechende Grafiksignale (direkt oder indirekt) an die grafischen Ausgabeeinheiten ausgeben. Die spezialisierte Fachkraft kann somit erkennen, ob die Anpassung des Untersuchungsprogramms vorteilhaft war und gegebenenfalls weitere Anpassungen direkt vornehmen.

Soweit nicht explizit angegeben ist die Reihenfolge der oben beschriebenen Verfahrensschritte nicht fest vorgegeben. Das erfindungsgemäße Verfahren kann zumindest teilweise durch ein Computerprogramm ausgeführt werden, umfassend entsprechende Instruktionen, um einen Computer zu veranlassen, einen oder mehrere der Verfahrensschritte auszuführen.

### 4. Beschreibung bevorzugter Ausführungsbeispiele

Nachfolgend wird die Erfindung unter Bezugnahme auf die beiliegenden Figuren näher beschrieben. Es zeigen:
Figur 1 ein System zur Durchführung einer bildgebenden diagnostischen Untersuchung gemäß einem Ausführungsbeispiel; und
Figur 2 ein Verfahren zur Durchführung einer bildgebenden diagnostischen Untersuchung gemäß einem weiteren Ausführungsbeispiel.

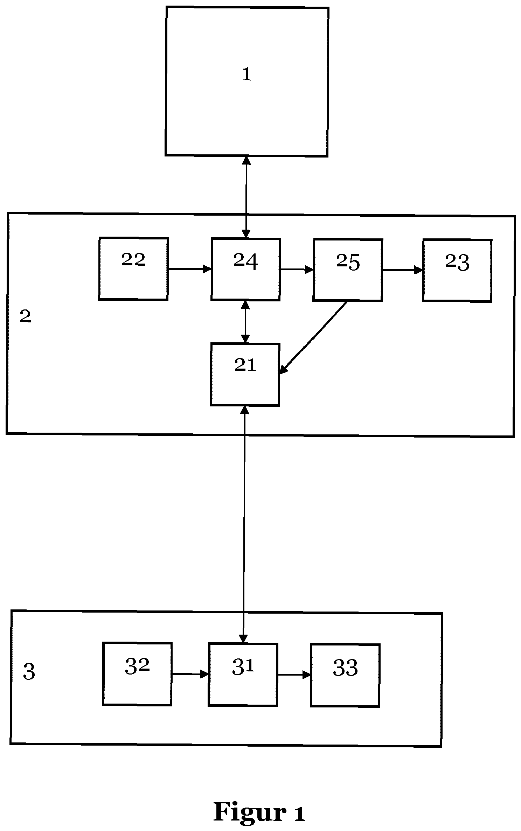

In der Figur 1 ist schematisch ein System zur Durchführung einer bildgebenden diagnostischen Untersuchung gemäß eines Ausführungsbeispiels dargestellt. Das System umfasst eine MRT-Untersuchungsvorrichtung 1, welche zur Erzeugung von diagnostischen Bilddaten dient. Hierzu umfasst diese eine Magnetresonanzanlage.

Ferner ist eine lokale Bedienvorrichtung 2 vorgesehen. Diese ist lokal bei der MRT-Untersuchungsvorrichtung 1 angeordnet. Die Bedienvorrichtung 2 umfasst eine Recheneinheit bzw. einen Steuerrechner 24, welcher mit der MRT-Unterersuchungsvorrichtung 1 in Kommunikation steht. Beispielsweise können der Steuerrechner 24 und die MRT-Unterersuchungsvorrichtung 1 über ein Netzwerk, wie etwa ein LAN miteinander kommunizieren. Hierzu sind entsprechende Netzwerkschnittstellen vorgesehen. Der Steuerrechner 24 umfasst dabei computerimplementierte Instruktionen, welche es erlauben, die MRT-Unterersuchungsvorrichtung 1 gemäß registrierten Nutzereingaben zu steuern. Der Steuerrechner 24 umfasst unter anderem eine CPU, eine Grafikkarte, eine Grafiksignal-Ausgabeschnittstelle und eine Eingabeschnittstelle.

Die lokale Bedienvorrichtung 2 umfasst ferner lokale Peripheriegeräte 22 (z.B. Maus und Tastatur), mittels welcher Eingaben von einem Nutzer registriert werden können. Die lokalen Peripheriegeräte sind dabei an die Eingabeschnittstelle des Steuerrechners 24 angeschlossen, beispielsweise über eine USB-Verbindung.

Eine Grafiksignal-Ausgabeschnittstelle des Steuerrechners 24 ist mit einem Videosplitter 25 verbunden. Die von dem Steuerrechner 24 ausgegebenen Grafik- oder Videosignale werden dabei durch den Videosplitter 25 gesplittet. Ein erster Ausgang des Videosplitters 25 ist mit einem lokalen Monitor 23 verbunden, auf welchem somit die von dem Steuerrechner 24 ausgegebenen Grafiksignale dargestellt werden. Ein zweiter Ausgang des Videosplitters 25 ist mit einer ersten Verbindungseinheit verbunden, welche als KVM-Transmitter 21 eines IP-basierten KVM-Extender- bzw. Verlängerungssystems (oder eines IP-basierten KVM-Verlängerung-und-Switching-Systems) ausgestaltet ist. Auch der Steuerrechner 24 ist über eine entsprechende Schnittstelle, beispielsweise eine USB-Schnittstelle, mit dem KVM-Transmitter 21 verbunden. Vorliegend sind der KVM-Transmitter 21 und der Videosplitter 25 als separate Einheiten vorgesehen. Allerdings können diese auch integral miteinander ausgebildet sein.

Neben der lokalen Bedienvorrichtung 2 ist ferner eine externe Bedienvorrichtung 3 vorgesehen, welche separat und entfernt von der lokalen Bedienvorrichtung 2 angeordnet ist. Diese externe Bedienvorrichtung 3 umfasst eine zweite Verbindungseinheit, welche als entsprechender KVM-Receiver 31 des KVM-Verlängerungssystems ausgestaltet ist. Die externe Bedienvorrichtung 3 umfasst ferner externe Peripheriegeräte 32 (z.B. Maus und Tastatur), die direkt an den KVM-Receiver 31 angeschlossen sind. Ferner ist ein externer Monitor 33 vorgesehen, welcher ebenfalls direkt an den KVM-Receiver 31 angeschlossen ist. Mittels des Videosplitters 25, des KVM-Transmitters 21 und des KVM-Receivers 31 können Grafiksignale somit auch an den externen Monitor 33 ausgegeben werden.

Eine Keyboard-Video-Mouse-(KVM)-Funktionalität ermöglicht es einem Nutzer, entfernten Computer mittels einer Remote-Tastatur, eines Remote-Monitors und/oder einer Remote-Maus zu steuern. Vorliegend ist der KVM-Transmitter 21 und der KVM-Receiver 31 zum gegenseitigen Datenaustausch über eine echtzeitfähige Datenverbindung eingerichtet. Der KVM-Transmitter 21 und der KVM-Receiver 31 umfassen hierfür entsprechende KVM-Verlängerungsmodule. Somit kann ein Nutzer softwareunabhängig von der externen Bedienvorrichtung 3 Eingaben an den Steuerrechner 24 tätigen, oder gleichermaßen von der lokalen Bedienvorrichtung 2. Zudem werden die von dem Steuerrechner 24 ausgegebenen Grafiksignale sowohl auf dem lokalen Monitor 23 und gleichfalls auch auf dem externen Monitor 33 angezeigt. Eine spezialisierte Fachkraft kann somit die Untersuchungsvorrichtung über die externe Bedienvorrichtung 3 in der gleichen Art steuern, wie sie es über die lokale Bedienvorrichtung 2 tun könnte. Letztlich werden dadurch eine unabhängige Fernanzeige und eine unabhängige Fernsteuerung ermöglicht.

Beispielsweise kann der KVM-Transmitter 21 ein "Emerald^{®} SE DVI IP-basierter KVM Extender Sender (Black Box EMD2000SE-T)" sein, und der KVM-Receiver 31 kann ein "DVI IP-basierter KVM Extender Empfänger (Black Box EMD2000SE-R)" sein.

Der KVM-Transmitter 21 (eines IP-basierten KVM-Verlängerung-und-Switching-Systems) kann dazu eingerichtet sein, um mit mehreren KVM-Receivern gekoppelt zu werden. Somit kann die MRT-Untersuchungsvorrichtung 1 durch die lokale Bedienvorrichtung 2 gesteuert werden, und auch durch mehrere externe Bedienvorrichtungen, die über den KVM-Transmitter 21 mit dem Steuerrechner 24 kommunizieren (nicht in der Figur 1 gezeigt). Eine weitere externe Bedienvorrichtung kann beispielsweise eine eigene externe Verbindungseinheit umfassen, welche in einem Softwaremodul implementiert sein kann (z.B. in einer App), und mit dem lokalen KVM-Transmitter 21 kommunizieren kann.

Gleichermaßen kann der KVM-Receiver 31 (eines IP-basierten KVM-Verlängerung-und-Switching-Systems) dazu eingerichtet sein, um mit mehreren KVM-Transmittern gekoppelt zu sein. Somit kann die externe Bedienvorrichtung 3 verwendet werden, um neben der MRT-Untersuchungsvorrichtung 1 auch weitere Untersuchungsvorrichtungen, wie etwa eine CT-Untersuchungsvorrichtung, zu steuern (nicht in der Figur 1 gezeigt).

Mittels der KVM-Komponenten 21, 31 und des Videosplitters 25 ist ein Aufrüsten bestehender Systeme mit geringem Aufwand möglich. Eingriffe in die Software des Steuerrechners 24 sind nicht notwendig.

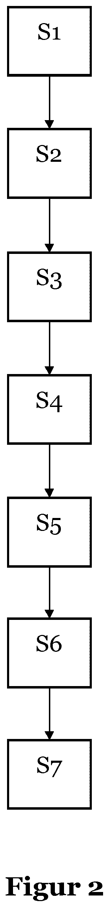

In der Figur 2 wird ein Ablaufdiagramm eines Verfahrens zur Durchführung einer bildgebenden diagnostischen Untersuchung gemäß einer Ausführungsform gezeigt. Dieses Verfahren wird nachfolgend anhand des Systems gemäß Figur 1 näher erläutert, ist aber nicht hierauf beschränkt.

Das Verfahren beginnt mit Schritt S1, bei dem ein System zur Durchführung einer bildgebenden diagnostischen Untersuchung bereitgestellt wird, welches dem System gemäß Figur 1 entsprechen kann. Ein Patient, an dem die Untersuchung vorgenommen werden soll, kann entsprechend der durchzuführenden Untersuchung von einer allgemeinen Hilfskraft vorbereitet werden und an der MRT-Unterersuchungsvorrichtung 1 positioniert werden.

In einem weiteren Schritt S2 wird eine Kommunikation zwischen der lokalen Bedienvorrichtung 2 und der externen Bedienvorrichtung 3 hergestellt. Diese Kommunikation erfolgt über ein Netzwerk (z.B. LAN oder WAN), indem der KVM-Transmitter 21 und der KVM-Receiver 31 über das Netzwerk eine entsprechende Verbindung aufbauen. Dies kann durch entsprechende Eingaben mit den lokalen Peripheriegeräten 22, und/oder durch entsprechende Eingaben mit den externen Peripheriegeräten 32 gesteuert werden.

In einem weiteren Schritt S3 werden von dem Steuerrechner 24 Patientendaten als grafische Signale ausgegeben. Diese werden durch den Videosplitter 25 gesplittet, und direkt auf dem lokalen Monitor 23 angezeigt. Zudem werden die Signale von dem Videosplitter auch an den KVM-Transmitter 21 gesendet, welcher die Signale an den KVM-Receiver 31 weiterleitet, sodass die Signale auch auf dem dort angeschlossenen externen Monitor 33 angezeigt werden. Somit kann die allgemeine Hilfskraft, die lokal den Patienten versorgt, die Patientendaten einsehen, aber auch eine spezialisierte Fachkraft, die extern die externe Bedienvorrichtung 3 bedient.

In einem weiteren Schritt S4 tätigt die spezialisierte Hilfskraft an der externen Bedienvorrichtung 3 Eingaben, mittels der externen Peripheriegeräte 32. Die entsprechenden Signale werden sodann über den KVM-Receiver 31 und den KVM-Transmitter 21 an den Steuerrechner 24 geleitet. Basierend auf diesen Eingaben wird durch den Steuerrechner 24 die MRT-Untersuchungsvorrichtung 1 angesteuert, um das entsprechende Untersuchungsprogramm durchzuführen. Zusätzlich kann auch die allgemeine Hilfskraft an der lokalen Bedienvorrichtung 2 Eingaben tätigen, beispielsweise Informationen bezüglich Kontrastmittel eingeben, die von ihr dem Patienten injiziert wurden. Nachfolgend startet die MRT-Untersuchungsvorrichtung 1 das Untersuchungsprogramm.

Während des Ablaufs des Untersuchungsprogramms erfolgt in einem Schritt S5 ein Darstellen von durch die MRT-Untersuchungsvorrichtung 1 erzeugte diagnostische Bilddaten auf dem lokalen Monitor 23 und auf dem externen Monitor 33. Hierfür werden die entsprechenden Grafiksignale, welche von dem Steuerrechner 24 ausgegeben werden, ebenfalls durch den Videosplitter 25 gesplittet und sodann an die beiden Monitore geleitet. Die spezialisierte Fachkraft an der externen Bedienvorrichtung kann somit während des Ablaufs des Untersuchungsprogramms die Ergebnisse sichten und beurteilen.

In einem weiteren Schritt S6 passt die spezialisierte Fachkraft das Untersuchungsprogramm an, um dieses angesichts der bisherigen Zwischenergebnisse und der diagnostischen Fragestellung zu optimieren. Beispielsweise kann ein Detaillierungsgrad der Aufnahmen angepasst werden, und der zu untersuchende Bereich des Patientenkörpers. Hierzu gibt sich über die externen Peripheriegeräte 32 entsprechende Befehle ein, welche über den KVM-Receiver 31 und den KVM-Transmitter 21 an den Steuerrechner 24 geleitet und von diesem entsprechend umgesetzt werden. Anschließend wird von der MRT-Untersuchungsvorrichtung 1 das entsprechend angepasste Untersuchungsprogramm durchgeführt.

In einem weiteren Schritt S7, welcher während des Durchführens des angepassten Untersuchungsprogramms erfolgt, werden die durch die MRT-Untersuchungsvorrichtung 1 erzeugten diagnostischen Bilddaten auf dem lokalen Monitor 23 und auf dem externen Monitor 33 dargestellt. Die spezialisierte Fachkraft kann an der externen Bedienvorrichtung 3 nun die Auswirkung der Anpassung des Untersuchungsprogramms überprüfen, und gegebenenfalls das Untersuchungsprogramm durch Wiederholung der Schritte S5 und S6 weiter optimieren. Die spezialisierte Fachkraft kann somit von extern die vollständige Steuerung der eigentlichen Untersuchung effizient durchführen.

In einem weiteren Ausführungsbeispiel kann im Rahmen der Durchführung einer bildgebenden diagnostischen Untersuchung einer oder mehrere der folgenden Schritte wahlweise in Kombination mit den zuvor beschriebenen Ausführungsbeispielen durchgeführt werden:
Herstellen einer echtzeitfähigen Datenverbindung zwischen dem KVM-Transmitter 21 und dem KVM-Receiver 31, wobei dies vorzugsweise durch die externe Bedienvorrichtung 3 erfolgt bzw. gesteuert wird. Abgreifen von Grafikausgabesignalen von der Grafiksignal-Ausgabeschnittstelle des Steuerrechners 24 durch den Videosplitter 25 und Weiterleiten dieser Signale an den lokalen Monitor 23 und den KVM-Transmitter 21. Übermitteln der Grafikausgabesignale von dem KVM-Transmitter 21 an den KVM-Receiver 31 und Weiterleiten dieser Signale von dem KVM-Receiver 31 an den externen Monitor 33. Anzeigen der Grafikausgabesignale durch den externen Monitor 33.

Ferner kann zum Starten eines Untersuchungsprogramms einer oder mehrere der folgenden Schritte wahlweise in Kombination mit den zuvor beschriebenen Ausführungsbeispielen durchgeführt werden:
Starten eines Untersuchungsprogramms durch die externen Peripheriegeräte 32 und Übermitteln eines entsprechenden Signals zum Starten des Untersuchungsprogramms an den KVM-Receiver 31. Übermitteln des Signals zum Starten des Untersuchungsprogramms von dem KVM-Receiver 31 an den KVM-Transmitter 21 und Weiterleiten dieses Signals von dem KVM-Transmitter 21 an den Steuerrechner 24 der lokalen Bedienvorrichtung 2. Steuern der MRT-Untersuchungsvorrichtung 1 durch den Steuerrechner 24 zur Erzeugung von Bilddaten anhand des Signals zum Starten des Untersuchungsprogramms. Übermitteln von Grafikausgabesignalen zur Anzeige eines Startbildes des Untersuchungsprogramms von der Grafiksignal-Ausgabeschnittstelle des Steuerrechners 24 an den Videosplitter 25 und Weiterleiten dieser Signale von dem Videosplitter 25 an den lokalen Monitor 23 und den KVM-Transmitter 21. Übermitteln der Signale zur Anzeige des Startbildes des Untersuchungsprogramms von dem KVM-Transmitter 21 an den KVM-Receiver 31 und Weiterleiten dieser Signale von dem KVM-Receiver 31 an den externen Monitor 33. Anzeigen des Startbildes des Untersuchungsprogramms durch den externen Monitor 33 (und durch den lokalen Monitor 23).

Ferner kann einer oder mehrere der folgenden Schritte wahlweise in Kombination mit den zuvor beschriebenen Ausführungsbeispielen durchgeführt werden:
Starten einer Messserie eines gewählten Untersuchungsprotokolls durch die externen Peripheriegeräte 32 und Übermitteln eines entsprechenden Signals zum Starten einer Messserie an den KVM-Receiver 31. Übermitteln des Signals zum Starten der Messserie von dem KVM-Receiver 31 an den KVM-Transmitter 21 und Weiterleiten dieses Signals von dem KVM-Transmitter 21 an den Steuerrechner 24 der lokalen Bedienvorrichtung 2. Steuern der MRT-Untersuchungsvorrichtung 1 durch den Steuerrechner 24 zum Starten der Messserie anhand des Signals zum Starten der Messserie. Aufnehmen der Messserie durch die MRT-Untersuchungsvorrichtung 1 und Erzeugen von Bilddaten und Übermitteln der erzeugten Bilddaten an den Steuerrechner 24. Übermitteln von Grafikausgabesignalen zur Anzeige der Bilddaten von der Grafiksignal-Ausgabeschnittstelle des Steuerrechners 24 an den Videosplitter 25 und Weiterleiten dieser Signale von dem Videosplitter 25 an den lokalen Monitor 23 und den KVM-Transmitter 21. Übermitteln der Grafikausgabesignale zur Anzeige der Bilddaten von dem KVM-Transmitter 21 an den KVM-Receiver 31 und Weiterleiten dieser Signale von dem KVM-Receiver 31 an den externen Monitor 33. Anzeigen der Bilddaten durch den externen Monitor 33 (und durch den lokalen Monitor 23). Auswählen eines Teilbereichs der angezeigten Bilddaten als Bilddaten-Erzeugungsbereich und/oder Anpassen von Eigenschaftsparametern für Folge-Bilddaten einer Folge-Messserie durch die externen Peripheriegeräte 32 und Übermitteln eines entsprechenden Signals an den KVM-Receiver 31. Übermitteln dieses Signals an den KVM-Transmitter 21 und Weiterleiten dieses Signals an den Steuerrechner 24. Steuern der MRT-Untersuchungsvorrichtung 1 durch den Steuerrechner 24 zum Auswählen eines Teilbereichs der angezeigten Bilddaten und/oder zum Anpassen von Eigenschaftsparametern für die Folge-Bilddaten einer Folge-Messserie anhand jenes Signals zum Auswählen eines Teilbereichs der angezeigten Bilddaten und/oder zum Anpassen von Eigenschaftsparametern für die Folge-Bilddaten einer Folge-Messserie. Aufnehmen einer Folge-Messserie und Erzeugen von Bilddaten durch die MRT-Untersuchungsvorrichtung 1 und Übermitteln der erzeugten Bilddaten an den Steuerrechner 24. Übermitteln von Grafikausgabesignalen zur Anzeige der Bilddaten von der Grafiksignal-Ausgabeschnittstelle des Steuerrechners 24 an den Videosplitter 25 und Weiterleiten dieser Signale von dem Videosplitter 25 an den lokalen Monitor 23 und den KVM-Transmitter 21. Übermitteln der Grafikausgabesignale zur Anzeige der Bilddaten von dem KVM-Transmitter 21 an den KVM-Receiver 31 und Weiterleiten dieser Signale von dem KVM-Receiver 31 an den externen Monitor 33. Anzeigen der Bilddaten durch den externen Monitor 33 (und durch den lokalen Monitor 23). Initiieren des Beendens des Untersuchungsprogramms durch die externen Peripheriegeräte 32 und Übermitteln eines entsprechenden Signals zum Beenden des Untersuchungsprogramms an den KVM-Receiver 31. Übermitteln des Signals von dem KVM-Receiver 31 an den KVM-Transmitter 21 und Weiterleiten des Signals an den Steuerrechner 24. Beenden des Untersuchungsprogramms durch den Steuerrechner 24.

## Patentansprüche

1. System zur Durchführung einer bildgebenden diagnostischen Untersuchung, aufweisend:
eine Untersuchungsvorrichtung (1), insbesondere eine MRT- oder CT-Untersuchungsvorrichtung, eingerichtet zur Erzeugung von diagnostischen Bilddaten;
eine lokale Bedienvorrichtung (2), eingerichtet zur Steuerung der Untersuchungsvorrichtung, umfassend
- eine lokale Eingabeeinheit (22), eingerichtet zur Registrierung von Nutzereingaben;
- eine lokale grafische Ausgabeeinheit (23), eingerichtet zur Darstellung der mit der Untersuchungsvorrichtung (1) erzeugten Bilddaten;
- eine lokale Verbindungseinheit (21);
- Steuermittel (24) zur Steuerung der Untersuchungsvorrichtung;
eine externe Bedienvorrichtung (3), die separat zu der lokalen Bedienvorrichtung (2) bereitgestellt ist, umfassend
- eine externe Eingabeeinheit (32), eingerichtet zur Registrierung von Nutzereingaben;
- eine externe grafische Ausgabeeinheit (33), eingerichtet zur Darstellung der mit der Untersuchungsvorrichtung (1) erzeugten Bilddaten;
- eine externe Verbindungseinheit (31);
wobei die lokale und externe Verbindungseinheit (21, 31) zur Kommunikation miteinander über ein Netzwerk eingerichtet sind;
wobei das System eingerichtet ist, um eine Steuerung der Untersuchungsvorrichtung (1) gemäß den durch die lokale Eingabeeinheit (22) registrierten Nutzereingaben in Echtzeit zu ermöglichen;
wobei das System eingerichtet ist, um die Steuerung der Untersuchungsvorrichtung (1) gemäß den durch die externe Eingabeeinheit (32) registrierten Nutzereingaben in Echtzeit zu ermöglichen.

2. System nach Anspruch 1, wobei die lokale Verbindungseinheit (21) und die externe Verbindungseinheit (31) KVM-Verlängerungsmodule, insbesondere IP-basierte KVM-Verlängerungsmodule umfassen.

3. System nach Anspruch 1 oder 2, wobei die lokale Verbindungseinheit (21) und die externe Verbindungseinheit (31) eingerichtet sind, um die durch die externe Eingabeeinheit (32) registrierten Nutzereingaben an die Steuermittel (24) zu übertragen, und wobei die lokale Verbindungseinheit (21) und die externe Verbindungseinheit (31) eingerichtet sind, um die mit der Untersuchungsvorrichtung (1) erzeugten Bilddaten an die externe grafische Ausgabeeinheit (33) zu übertragen.

4. System nach einem der Ansprüche 1 bis 3, wobei die externe Eingabeeinheit (32) und die externe grafische Ausgabeeinheit (33) direkt mit der externen Verbindungseinheit (31) verbunden sind.

5. System nach einem der Ansprüche 1 bis 4, wobei die lokale Bedienvorrichtung (2) ferner einen Videosplitter (25) umfasst, eingerichtet um ein eingehendes Videosignal zu splitten, wobei das gesplittete Videosignal an die lokale grafische Ausgabeeinheit (23) und über die lokale Verbindungseinheit (21) und die externe Verbindungseinheit (31) an die externe grafische Ausgabeeinheit (33) ausgegeben wird.

6. System nach einem der Ansprüche 1 bis 5, wobei die Steuermittel zumindest teilweise durch eine Recheneinheit (24) ausgebildet sind, welche eingerichtet ist zum Ausgeben von Steuersignalen gemäß den registrierten Nutzereingaben an die Untersuchungsvorrichtung (1), zum Empfangen der erzeugten diagnostischen Bilddaten von der Untersuchungsvorrichtung (1), und zur Ausgabe der diagnostischen Bilddaten.

7. System nach Anspruch 6 in Kombination mit Anspruch 4, wobei die Recheneinheit (24) eingerichtet ist, die diagnostischen Bilddaten an den Videosplitter (25) auszugeben.

8. System nach Anspruch 6 oder 7, wobei die Recheneinheit (24) eingerichtet ist, um über die externe Eingabeeinheit (32) unabhängig von der lokalen Eingabeeinheit (22) bedient zu werden, und wobei die Recheneinheit (24) eingerichtet ist, um über die lokale Eingabeeinheit (22) unabhängig von der externen Eingabeeinheit (32) bedient zu werden.

9. System nach einem der Ansprüche 6 bis 8, wobei das System eingerichtet ist, um eine Bedienung der Recheneinheit (24) ab Bootvorgang über die externe Eingabeeinheit (32) und über die lokale Eingabeeinheit (22) zu ermöglichen.

10. System nach einem der Ansprüche 1 bis 9, wobei das System eingerichtet ist, um eine Steuerung der Untersuchungsvorrichtung (1) mittels der externen Bedienvorrichtung (3) vor, während und nach der Erzeugung der diagnostischen Bilddaten zu ermöglichen.

11. System nach einem der Ansprüche 1 bis 10, ferner umfassend:
eine zweite Untersuchungsvorrichtung, eingerichtet zur Erzeugung von diagnostischen Bilddaten;
eine zweite lokale Bedienvorrichtung, eingerichtet zur Steuerung der zweiten Untersuchungsvorrichtung, umfassend
- eine zweite lokale Eingabeeinheit, eingerichtet zur Registrierung von Nutzereingaben;
- eine zweite lokale grafische Ausgabeeinheit, eingerichtet zur Darstellung der mit der zweiten Untersuchungsvorrichtung erzeugten Bilddaten;
- eine zweite lokale Verbindungseinheit;
- zweite Steuermittel zur Steuerung der zweiten Untersuchungsvorrichtung;
wobei die zweite lokale Verbindungseinheit und die externe Verbindungseinheit (31) zur Kommunikation miteinander über das Netzwerk eingerichtet sind;
wobei das eingerichtet ist, um die Steuerung der zweiten Untersuchungsvorrichtung gemäß den durch die zweite lokale Eingabeeinheit registrierten Nutzereingaben in Echtzeit zu ermöglichen,
wobei das System eingerichtet ist, um die Steuerung der zweiten Untersuchungsvorrichtung gemäß den durch die externe Eingabeeinheit registrierten Nutzereingaben in Echtzeit zu ermöglichen.

12. Verfahren zur Durchführung einer bildgebenden diagnostischen Untersuchung, umfassend:
Bereitstellen eines Systems gemäß einem der Ansprüche 1 bis 11;
Herstellen einer Kommunikation zwischen der lokalen Bedienvorrichtung (2) und der externen Bedienvorrichtung (3) über die lokale Verbindungseinheit (21) und die externe Verbindungseinheit (31);
Darstellen von Patientendaten auf der lokalen grafischen Ausgabeeinheit (23) und auf der externen grafischen Ausgabeeinheit (33);
Ansteuern der Untersuchungsvorrichtung (1) zum Durchführen eines Untersuchungsprogramms zur Erzeugung von diagnostischen Bilddaten, basierend auf durch die lokale Eingabeeinheit (22) und/oder durch die externe Eingabeeinheit (32) registrierten Nutzereingaben;
während des Durchführens des Untersuchungsprogramms: Darstellen von erzeugten diagnostischen Bilddaten auf der lokalen grafischen Ausgabeeinheit (23) und auf der externen grafischen Ausgabeeinheit (33);
anschließend, während des Durchführens des Untersuchungsprogramms: Anpassen des Untersuchungsprogramms, basierend auf durch die lokale Eingabeeinheit (22) und/oder durch die externe Eingabeeinheit (32) registrierten Nutzereingaben;
während des Durchführens des angepassten Untersuchungsprogramms: Darstellen von erzeugten diagnostischen Bilddaten auf der lokalen grafischen Ausgabeeinheit (23) und auf der externen grafischen Ausgabeeinheit (33).
